# FASCICULE DE BREVET EUROPEEN

(11) **EP 3 482 788 B1**
(45) Date de publication et mention de la délivrance du brevet: **17.02.2021**
(21) Numéro de dépôt: 18198811.4
(22) Date de dépôt: 05.10.2018
(51) Int. Cl.: A61M 16/00, F04D 29/08, F04D 29/42, F04D 29/60, F04D 29/66, H02K 5/24

(54) **APPAREIL D'ASSISTANCE RESPIRATOIRE À MICRO-SOUFFLANTE MOTORISÉE**
GERÄT ZUR ATMUNGSUNTERSTÜTZUNG MIT MOTORISIERTEM MIKROGEBLÄSE
BREATHING ASSISTANCE APPARATUS WITH MOTORISED MICRO-FAN

(30) Priorité: 10.11.2017 FR 1760571
(43) Date de publication de la demande: 15.05.2019
(73) Titulaire: Air Liquide Medical Systems, 92160 Antony (FR)
(72) Inventeur: DAVOINE, Romain, 75014 PARIS (FR)
(74) Mandataire: Air Liquide

(56) Documents cités:
- WO-A1-2012/139681
- WO-A1-2013/133889
- FR-A1- 2 953 142
- FR-A1- 3 001 155
- US-A1- 2007 007 271

## Description

L'invention concerne un appareil d'assistance respiratoire comprenant une micro-soufflante motorisée maintenue en position dans la carcasse de l'appareil par des éléments amortisseurs.

Afin d'assister certains patients dans leur fonction respiratoire, on utilise des appareils d'assistance respiratoire ou de ventilation assistée, aussi appelés « ventilateurs » médicaux, qui délivrent un gaz respiratoire à un débit non nul et/ou à une pression supérieure à la pression atmosphérique (i.e. > 1 atm), typiquement de l'air (env. 21% d'O₂ en vol.) ou de l'air enrichi en oxygène (>21% d'O₂ en vol.).

Pour ce faire, on utilise des appareils d'assistance respiratoire mettant en œuvre une micro-soufflante, parfois simplement appelée « soufflante », « compresseur » ou «turbine », servant à aspirer l'air ambiant et à le délivrer à une pression donnée aux patients. Les documents EP-A-2165078, EP-A-2102504 et WO-A-2012/139681 décrivent de tels ventilateurs à micro-soufflante.

Classiquement, l'aspiration de l'air par la micro-soufflante se fait grâce à une (ou plusieurs) roue à ailettes agencée sur un arbre-moteur rotatif entraîné en rotation par un moteur électrique. La roue de la micro-soufflante est généralement agencée dans un compartiment interne aménagé dans une volute, typiquement formée d'une demi-volute inférieure et d'une demi-volute supérieure fixées l'une à l'autre et définissant entre elles un volume creux, c'est-à-dire un compartiment interne, au sein duquel peut tourner la roue. La roue est donc prise en « sandwich » entre les volutes inférieure et supérieure.

La micro-soufflante est agencée dans la carcasse ou coque de l'appareil qui sert non seulement à la maintenir et à la protéger contre les chocs éventuels, par exemple en cas de chute de l'appareil sur le sol, mais qui assure aussi un rôle d'atténuation acoustique du bruit généré par celle-ci. En effet, les ventilateurs destinés à traiter des pathologies du sommeil, telle l'apnée du sommeil, doivent être particulièrement silencieux pour ne pas gêner le patient ou son conjoint, pendant son/leur sommeil.

De même, le maintien de la micro-soufflante dans la carcasse doit être correct car sinon il peut s'ensuivre des défauts d'étanchéité gazeuse et/ou des détériorations de la micro-soufflante en cas de chocs, de chute et/ou de vibrations.

Or, les performances de beaucoup de ventilateurs actuels sont insuffisantes tant au plan acoustique qu'en termes de bon maintien de la micro-soufflante dans la carcasse.

De plus, certains ventilateurs médicaux ont une architecture complexe qui complique leur montage en usine et augmente négativement leur coût, notamment lors des opérations d'entretien et de maintenance nécessitant un montage/démontage de certains composants du ventilateur, en particulier de la micro-soufflante.

Le problème qui se pose est de proposer un appareil d'assistance respiratoire, c'est-à-dire un ventilateur médical, dont l'architecture a été améliorée pour garantir non seulement un montage facile et donc peu onéreux de la micro-soufflante dans la carcasse de l'appareil mais aussi une bonne étanchéité gazeuse et un bon maintien de la micro-soufflante dans la carcasse de l'appareil.

La solution de l'invention concerne alors un appareil d'assistance respiratoire, c'est-à-dire un ventilateur médical, comprenant un boitier comprenant une micro-soufflante comprenant un moteur électrique à arbre-moteur rotatif, agencé dans un carter, l'arbre-moteur portant au moins une roue à ailettes logée dans un compartiment interne situé dans une volute, ledit compartiment interne comprenant une entrée de gaz et une sortie de gaz, la sortie de gaz étant en communication fluidique avec un conduit de sortie de gaz, ledit appareil comprenant en outre :
- un premier élément amortisseur en matériau souple, c'est-à-dire flexible et/ou élastiquement déformable, agencé autour et au contact du carter du moteur en assurant une étanchéité fluidique entre eux, et
- un deuxième élément amortisseur en matériau souple, c'est-à-dire flexible et/ou élastiquement déformable, agencé autour et au contact du conduit de sortie en assurant une étanchéité fluidique entre eux,
et dans lequel :
- le deuxième élément amortisseur vient prendre appui sur le premier élément amortisseur en assurant une étanchéité fluidique entre eux, et
- la micro-soufflante est maintenue en position fixe dans le boitier par lesdits premier et deuxième éléments amortisseurs.

Selon le cas, l'appareil d'assistance respiratoire, c'est-à-dire le ventilateur médical, de l'invention peut comprendre l'une ou plusieurs des caractéristiques techniques suivantes :
- le premier élément amortisseur et/ou le deuxième élément amortisseur sont en matériau élastomère, notamment en élastomère thermoplastique, de préférence en silicone.
- la micro-soufflante est maintenue en suspension dans le boitier par les premier et deuxième éléments amortisseurs.
- le boitier est formé d'au moins deux demi-coques, c'est-à-dire d'au moins deux parties, venant se raccorder l'une à l'autre en prenant en sandwich ledit premier et/ou ledit deuxième élément amortisseur.
- le boitier forme une carcasse rigide autour de la micro-soufflante.
- le boitier est en thermoplastique, par exemple en ABS ou ABS-PC.
- le premier élément amortisseur est agencé de manière à assurer une étanchéité fluidique entre les deux demi-coques du boitier.
- le premier élément amortisseur s'étend entre le carter et la jonction des deux demi-coques du boitier, c'est-à-dire la zone ou ligne située à l'intersection des deux demi-coques du boitier lorsqu'elles sont assemblées l'une à l'autre.
- le deuxième élément amortisseur est pris en sandwich entre l'une des deux demi-coques du boitier et le premier élément amortisseur en assurant une étanchéité fluidique entre eux.
- la micro-soufflante est maintenue en position fixe dans le boitier uniquement par lesdits premier et deuxième éléments amortisseurs, notamment maintenu en suspension dans le boitier par lesdits premier et deuxième éléments amortisseurs.
- le premier élément amortisseur est formé d'une pièce unique comprenant plusieurs ouvertures.
- le premier élément amortisseur est formé d'une pièce unique comprenant plusieurs ouvertures dont une ouverture de section circulaire conformée pour venir épouser le profil externe du carter du moteur de la micro-soufflante.
- le deuxième élément amortisseur est formé d'une pièce unique comprenant une ouverture centrale de section circulaire.
- le deuxième élément amortisseur comprend deux parois tubulaires agencées de manière concentrique, i.e. coaxiales l'une à l'autre, et réunies par une paroi de jonction de sorte de définir un espacement inter-parois à fond borgne entre lesdites parois tubulaires.
- le deuxième élément amortisseur comprend également un ou plusieurs aménagements de paroi réalisés dans ou portés par sa paroi périphérique externe, par exemple un ou plusieurs évidements, notamment des gorges ou analogues, ou un ou plusieurs épaulements.
- le ou les aménagements de paroi du deuxième élément amortisseur coopèrent avec des structures en relief, tels des parois, des doigts ou analogues, portées par le boitier et/ou par le premier élément amortisseur, lesdites structures en relief venant se loger dans le ou les aménagements de paroi et/ou le ou les épaulements venant prendre appui sur des structures en relief, telles des parois du boitier.
- il comprend en outre au moins une butée antichoc agencée entre la volute et l'une des deux demi-coques du boitier, de préférence plusieurs butées antichoc, typiquement de 2 à 9 butées.
- au moins une butée antichoc est en matériau élastomère, notamment en élastomère thermoplastique, de préférence en silicone.
- la volute de la micro-soufflante est surmontée d'un pavillon comprenant un passage central en communication fluidique avec l'entrée de gaz du compartiment interne de la volute, la ou lesdites butées antichoc étant agencées entre l'une des deux demi-coques du boitier et ledit pavillon.
- la volute est formée d'au moins deux demi-volutes, à savoir une demi-volute supérieure et une demi-volute inférieure, assemblées l'une avec l'autre pour former le compartiment interne.
- la demi-volute supérieure est traversée par l'orifice d'entrée de gaz débouchant dans le compartiment interne.
- la demi-volute inférieure est traversée par l'arbre-moteur rotatif du moteur.
- la demi-volute supérieure et/ou la demi-volute inférieure sont en thermoplastique, par exemple en ABS.
- le conduit de sortie de gaz est solidaire de la demi-volute supérieure et/ou la demi-volute inférieure.
- le moteur électrique de la micro-soufflante est alimenté en courant électrique par un ou des câbles électriques.
- il comprend des moyens de pilotage commandant le moteur électrique de la micro-soufflante, telles des sondes de position rotor/stator et thermique pour assurer la sécurité.
- les moyens de pilotage et d'alimentation comprennent une carte électronique à microprocesseur et en outre des composants électroniques de puissance.
- il comprend un afficheur d'information, tel un écran digital ou analogue.
- il comprend une ou des touches, boutons, curseurs ou analogue, notamment un bouton arrêt/marche.

L'invention porte aussi sur une installation de distribution de gaz comprenant un appareil d'assistance respiratoire selon l'invention raccordé fluidiquement à une interface respiratoire, tel un masque respiratoire ou analogue, par l'intermédiaire d'un tuyau flexible.

L'invention va maintenant être mieux comprise grâce à la description détaillée suivante faite en références aux Figures annexées parmi lesquelles :
- la Figure 1 à 5 représentent différentes vues d'un appareil d'assistance respiratoire selon la présente invention et
- la Figure 6 à 8 représentent des vues en coupe de la micro-soufflante intégrée à un appareil d'assistance respiratoire selon la présente invention.

Les Figures ci-jointes représentent différentes vues (éclatées, assemblées, en coupe etc...) d'un appareil d'assistance respiratoire, c'est-à-dire d'un ventilateur médical, conforme à la présente invention intégrant une micro-soufflante 1 délivrant du gaz respiratoire, tel de l'air sous pression, à destination d'un patient.

Cette micro-soufflante 1 comprend un moteur 2 alimenté en courant électrique par des câbles électrique 17 ou analogue, et délimité par un carter 4 ou boitier de moteur, ici de forme cylindrique, définissant un compartiment ou volume interne, encore appelé compartiment-moteur, renfermant le moteur 2, un arbre rotatif 3, des bobines/aimants et les autres éléments constitutifs du moteur 2. Le moteur 2 de la micro-soufflante 1 est alimenté en courant électrique et est par ailleurs piloté, via des câbles électriques 17 ou analogues, venant se raccorder à l'extrémité inférieure du corps de moteur 2. Le courant électrique est issu d'une source de courant électrique, tel le secteur électrique, une ou plusieurs batteries ou toute autre source de courant électrique.

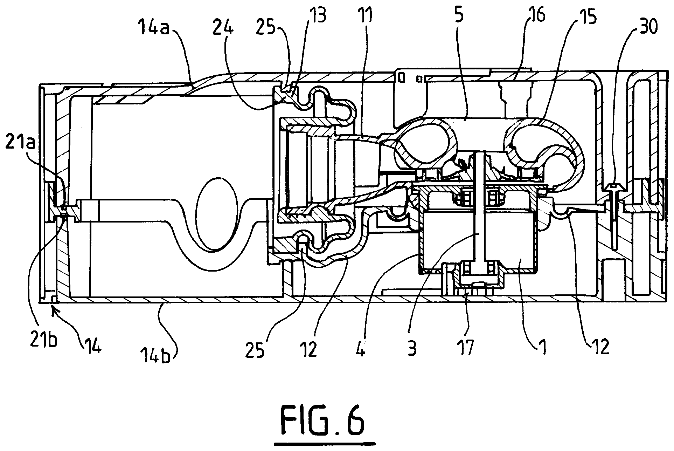

Comme visible sur les Figures 6 à 8, l'arbre-moteur 3 rotatif du moteur 2 fait saillie hors du moteur 2 à son extrémité supérieure en traversant un orifice aménagé dans la face supérieure du carter 4 du moteur 2. L'arbre-moteur 3 porte une roue à ailettes 5 et l'entraîne en rotation, pendant le fonctionnement du moteur 2, ce qui permet d'aspirer de l'air au sein du compartiment interne 6 dans lequel est agencée la roue 5, lequel est aménagé dans la volute 7 qui est elle-même formée entre deux demi-volutes 8a, 8b appelées volutes supérieure 8a et inférieure 8b. La volute 7 surmonte le carter 4 du moteur 2.

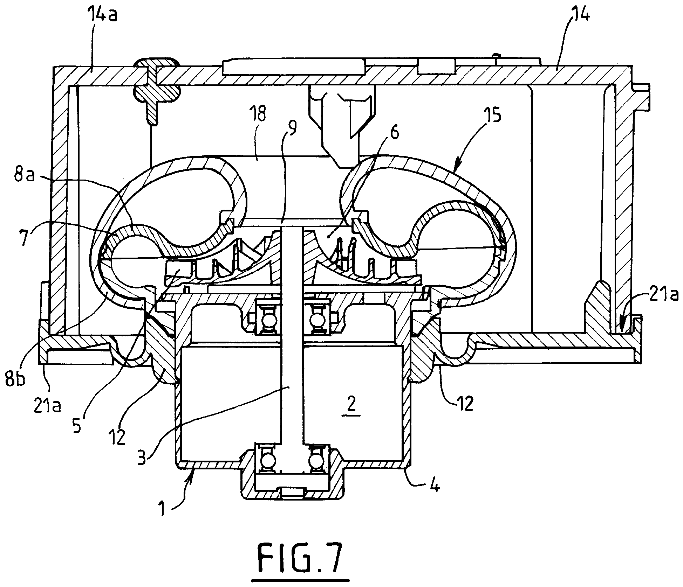

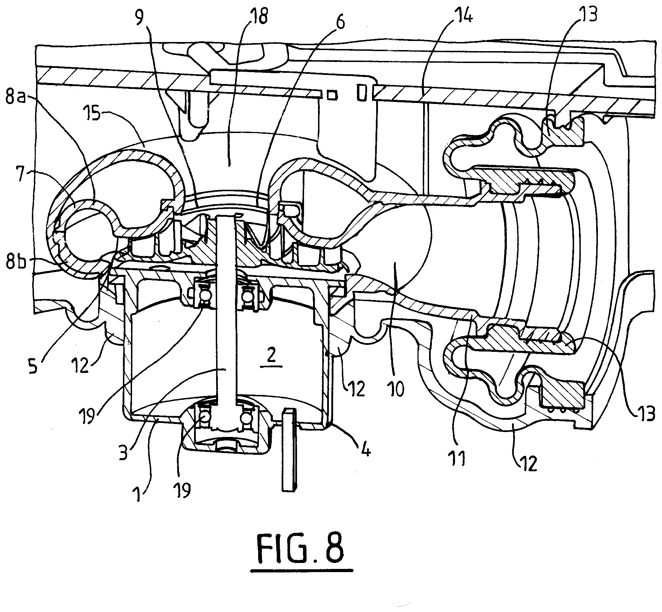

En fonctionnement, l'air aspiré par la roue à ailettes 5, pénètre dans le compartiment interne 6 situé de la volute 7 en traversant successivement le passage central 18 d'un pavillon 15 surmontant la volute 7 et l'ouverture 9 aménagée dans la volute supérieure 8a. Le passage central 18 du pavillon 15 et l'ouverture 9 de la volute supérieure 8a sont agencés en regard l'un de l'autre et en communication fluidique l'un avec l'autre, comme illustré sur les Figures 7 et 8. L'air aspiré lors des rotations de la roue à ailettes 5 est ensuite expulsé du compartiment interne 6 par un orifice de sortie 10 de gaz débouchant dans un conduit de sortie 11 permettant de véhiculer l'air qui est ensuite envoyé vers le patient, via un conduit flexible ou analogue (non montré).

La face inférieure de la roue 5 à ailettes est espacée et sensiblement parallèle à la surface supérieure externe du carter 4 du moteur 2, alors que la face supérieure de la roue 5 porte les ailettes. En général, la roue 5 est faite en matériau léger, typiquement en thermoplastique, tel du PEEK.

Habituellement, l'arbre-moteur 3 est maintenu et/ou guidé, lors de ses rotations, par des dispositifs à roulements 19 lubrifiés par des lubrifiants de type huile ou analogue. De préférence, des dispositifs à roulements sont agencés de part et d'autre du moteur 2, autour de l'axe 3.

Afin de garantir non seulement un montage facile et peu onéreux de la micro-soufflante 1 mais aussi une bonne étanchéité gazeuse et un bon maintien de la micro-soufflante 1 dans la carcasse 14 de l'appareil, selon la présente invention, on prévoit plusieurs éléments amortisseurs 12, 13 en matériau souple, typiquement en élastomère, telle de la silicone, agencés autour de plusieurs parties de la micro-soufflante 1.

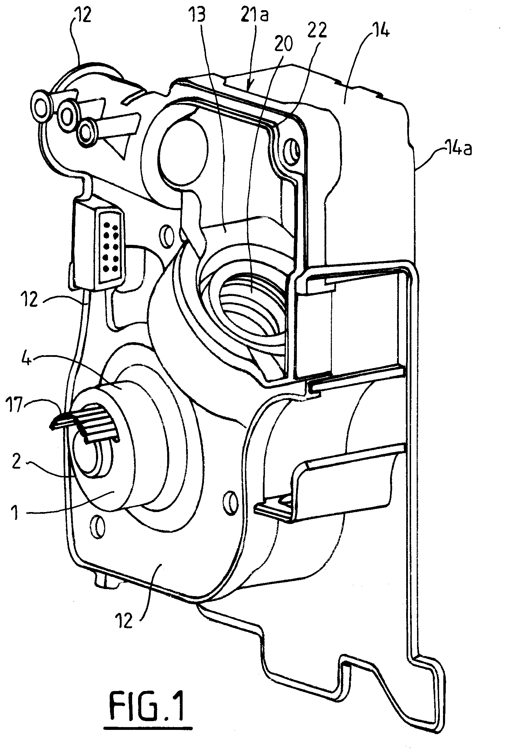

Ces éléments amortisseurs 12, 13 et leur agencement autour des parties de la micro-soufflante 1 sont illustrés en Figures 1 à 3. Comme on le voit, un premier élément amortisseur 12 en matériau souple est agencé autour et au contact du carter 4 du moteur 2 de sorte d'assurer une étanchéité fluidique entre eux, et un deuxième élément amortisseur 13 en matériau souple est, quant à lui, agencé autour et au contact du conduit de sortie 11 en assurant, là encore, une étanchéité fluidique entre eux. Ainsi que montré en Figure 8, le deuxième élément amortisseur 13 vient prendre appui sur le premier élément amortisseur 12 en assurant une étanchéité fluidique entre eux.

Ces éléments amortisseurs possèdent une dureté de l'ordre de 40 à 80 Shores A, une épaisseur de 0,5 à 3 mm, garantissant le bon découplage mécanique afin d'atténuer chocs/chutes/vibrations. L'amortisseur 13 doit idéalement être plus souple que l'amortisseur 12 afin d'assurer l'ensemble des écrasements.

Par ailleurs, la micro-soufflante 1 est maintenue en position fixe et stable dans le boitier ou carcasse 14 rigide de l'appareil par les éléments amortisseurs 12,13, sans qu'il soit nécessaire de prévoir d'autres éléments de soutien ou de suspension de la micro-soufflante 1, ce qui facilite grandement son montage et réduit son coût. Typiquement, le boitier ou carcasse 14 rigide de l'appareil est réalisé en polymère, thermoplastique, par exemple en ABS ou ABS-PC, de 1 à 3 mm d'épaisseur.

En fait, comme illustré sur les Figures 1 à 3, le premier élément amortisseur 12 s'étend depuis le carter 4 du moteur 2 jusqu'à la jonction des deux demi-coques 14a, 14b du boitier 14 de l'appareil, c'est-à-dire les rebords 21a, 21b des deux demi-coques 14a, 14b (cf. Fig. 6), alors que le deuxième élément amortisseur 13 est pris en sandwich entre l'une des deux demi-coques 14a du boitier 14 et le premier élément amortisseur 12 (cf. Fig. 1). Le premier élément amortisseur 12 constitue donc un joint d'étanchéité 22 entre les rebords 21a, 21b du boitier 14, au niveau d'au moins une partie de la zone de jonction des deux demi-coques 14a, 14b du boitier 14 de l'appareil, comme représenté sur la Figure 1.

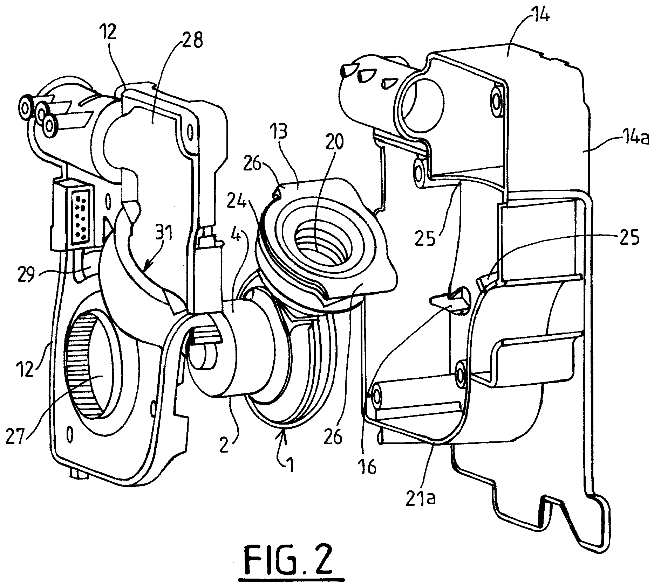

Le deuxième élément amortisseur 13 est, dans le mode de réalisation représenté, formé d'une pièce unique ayant une forme générale sensiblement tubulaire (cf. Fig. 2 à 5) comprenant une ouverture centrale 20, c'est-à-dire qu'elle est traversée par un passage central, dans laquelle vient se loger le conduit de sortie 11 en assurant une étanchéité fluidique et un bon maintien autour du conduit de sortie 11 puisque l'ouverture centrale 20 est en contact mécanique étroit avec la paroi périphérique externe du conduit de sortie 11, c'est-à-dire que le deuxième élément amortisseur 13 vient serrer élastiquement la paroi périphérique externe du conduit de sortie 11.

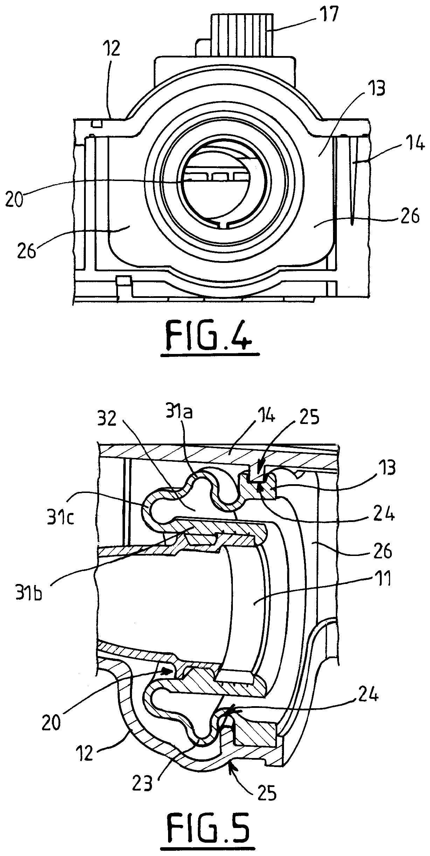

Plus précisément, le deuxième élément amortisseur 13 comprend en outre ici (cf. Figure 5) deux parois tubulaires 31a, 31b agencées de manière concentrique, i.e. coaxiales l'une à l'autre, et réunies à l'une de leurs extrémités par une paroi de jonction 31c de sorte de définir un espacement inter-parois 32 à fond borgne entre lesdites parois tubulaires 31a, 31b. Cette forme en « accordéon », telle une membrane, assure le bon découplage mécanique, c'est-à-dire de la mobilité et de la stabilité, entre éléments.

Le deuxième élément amortisseur 13 comprend également un ou plusieurs aménagements de paroi 24, 26, réalisés dans ou portés par sa paroi périphérique externe 23, par exemple un ou plusieurs évidements, notamment des gorges ou analogues, ou un ou plusieurs épaulements 26 coopérant avec des structures en relief 25, tels des parois, des doigts ou analogues, portées par le boitier 14 ou par le premier élément amortisseur 12 afin d'assurer un bon maintien en position du deuxième élément amortisseur 13, lesdites structures en relief 25 venant se loger dans les aménagements de paroi 24, comme détaillé en Figure 5, ou le ou les épaulements 26 venant prendre appui sur des structures en relief 25, telles des parois du boitier 14.

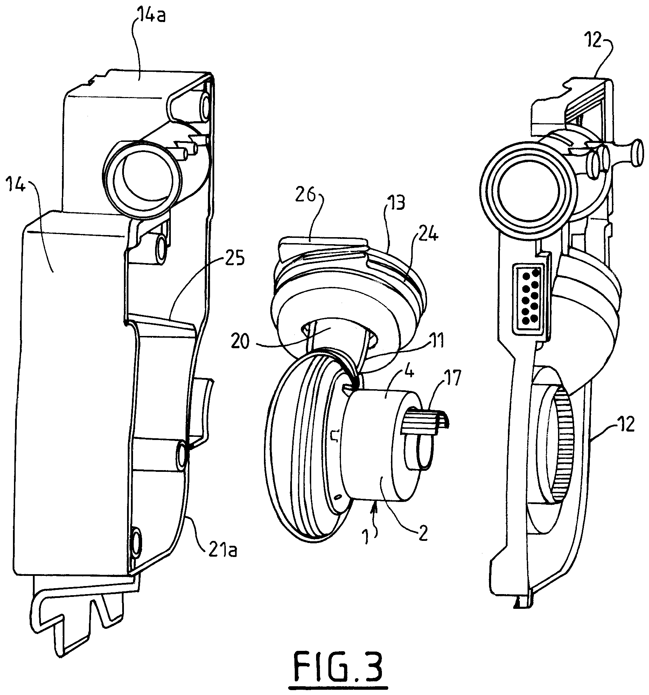

Par ailleurs, le premier élément amortisseur 12 est, quant à lui, dans le mode de réalisation représenté, formé d'une pièce unique ayant une forme générale complexe, comme illustré en Figure 2, s'étendant entre le carter 4 du moteur 2 et les rebords 21a, 21b des deux demi-coques 14a, 14b (cf. Fig. 6), comme déjà expliqué. Le premier élément amortisseur 12 est traversé de plusieurs ouvertures 27, 28,29 dont l'une 27 à une section circulaire puisque destinée à recevoir le carter 4 du moteur 2 qui est lui de forme cylindrique, comme illustré en figure 3.

Là encore, une étanchéité fluidique et un bon maintien autour du carter 4 sont garantis puisque l'ouverture à section circulaire 27 vient en contact mécanique étroit avec la paroi périphérique externe du carter 4, c'est-à-dire que le premier élément amortisseur 12 vient lui aussi serrer élastiquement, de l'ordre de 0,5 à 2 mm, la paroi périphérique externe du carter 4.

Par ailleurs, une partie 31 du premier élément amortisseur 12 est aussi conformée pour venir épouser le profil externe, c'est-à-dire la forme, du deuxième élément amortisseur 13 de manière à bien de maintenir et à assurer une étanchéité fluidique entre eux.

Le premier élément amortisseur 12 et les deux demi-coques 14a, 14b du boitier 14 de l'appareil sont maintenus solidaires par des éléments de jonction 30, telles des vis ou autres comme illustré en Fig. 6.

En fait, la micro-soufflante 1 est maintenue en position fixe et en suspension dans le boitier 14 de l'appareil, uniquement par lesdits premier et deuxième éléments amortisseurs 12, 13.

Préférentiellement, l'appareil de l'invention comprend aussi plusieurs butées 16 antichoc agencées entre la volute 7 et l'une 14a des deux demi-coques du boitier 14 de manière à préserver la micro-soufflante 1 en cas de choc, notamment de chute de l'appareil. Typiquement, on prévoit de 2 à 9 butées. Les butées 16 sont en élastomère, thermoplastique ou thermodurcissable, telle de la silicone, d'une dureté de l'ordre de 40 à 60 Shores A, et d'environ 0,5 à 2 mm d'épaisseur.

D'une façon générale, l'appareil de l'invention équipé de la micro-soufflante 1 maintenue par les éléments amortisseurs 12, 13 décrits ci-avant peut être utilisé pour traiter des pathologies respiratoires de tout type, en particulier apnée du sommeil, broncho-pneumopathie chronique obstructive (BPCO), troubles liés à l'obésité...

## Revendications

1. Appareil d'assistance respiratoire comprenant un boitier (14) comprenant une micro-soufflante (1) comprenant un moteur électrique (2) à arbre-moteur rotatif (3), agencé dans un carter (4), l'arbre-moteur (3) portant au moins une roue à ailettes (5) logée dans un compartiment interne (6) situé dans une volute (7), ledit compartiment interne (6) comprenant une entrée de gaz (9) et une sortie de gaz (10), la sortie de gaz (10) étant en communication fluidique avec un conduit de sortie (11) de gaz, ledit appareil comprenant en outre un premier élément amortisseur (12) en matériau souple, agencé autour et au contact du carter (4) du moteur (2) en assurant une étanchéité fluidique entre eux, et un deuxième élément amortisseur (13) en matériau souple, agencé autour et au contact du conduit de sortie (11) en assurant une étanchéité fluidique entre eux, et **caractérisé en ce que** le deuxième élément amortisseur (13) vient prendre appui sur le premier élément amortisseur (12) en assurant une étanchéité fluidique entre eux, et la micro-soufflante (1) est maintenue en position fixe dans le boitier (14) par lesdits premier et deuxième éléments amortisseurs (12, 13).

2. Appareil selon la revendication 1, **caractérisé en ce que** le premier élément amortisseur (12) et/ou le deuxième élément amortisseur (13) sont en matériau élastomère, de préférence en silicone.

3. Appareil selon l'une des revendications précédentes, **caractérisé en ce que** le boitier (14) est formé d'au moins deux demi-coques (14a, 14b) venant se raccorder l'une à l'autre en prenant en sandwich ledit premier et/ou ledit deuxième élément amortisseur (12,13).

4. Appareil selon la revendication 3, **caractérisé en ce que** le premier élément amortisseur (12) est agencé de manière à assurer une étanchéité fluidique entre les deux demi-coques (14a, 14b) du boitier (14).

5. Appareil selon l'une des revendications 3 ou 4, **caractérisé en ce que** le deuxième élément amortisseur (13) est pris en sandwich entre l'une des deux demi-coques (14a) du boitier (14) et le premier élément amortisseur (12) en assurant une étanchéité fluidique entre eux.

6. Appareil selon l'une des revendications 3 à 5, **caractérisé en ce que** la micro-soufflante (1) est maintenue en suspension dans le boitier par les premier et deuxième éléments amortisseurs, de préférence uniquement par lesdits premier et deuxième éléments amortisseurs (12, 13).

7. Appareil selon l'une des revendications 3 à 6, **caractérisé en ce que** le deuxième élément amortisseur (13) est formé d'une pièce unique comprenant une ouverture centrale (20) de section circulaire, et/ou le premier élément amortisseur (12) est formé d'une pièce unique comprenant plusieurs ouvertures (27, 28, 29).

8. Appareil selon l'une des revendications 3 à 7, **caractérisé en ce que** le premier élément amortisseur (12) s'étend entre le carter (4) et la jonction des deux demi-coques (14a, 14b) du boitier (14).

9. Appareil selon l'une des revendications 3 à 8, **caractérisé en ce qu'**il comprend en outre au moins une butée antichoc (16) agencée entre la volute (7) et l'une des deux demi-coques (14a) du boitier (14), de préférence plusieurs butées antichoc (16).

10. Appareil selon la revendication 9, **caractérisé en ce que** la volute (7) de la micro-soufflante (1) est surmonté d'un pavillon (15) comprenant un passage central (17) en communication fluidique avec l'entrée de gaz (9) du compartiment interne (6) de la volute (7), la ou lesdites butées antichoc (16) étant agencées entre l'une des deux demi-coques (14a) du boitier (14) et ledit pavillon (15).

11. Installation de distribution de gaz comprenant un appareil d'assistance respiratoire selon l'une des revendications précédentes raccordé fluidiquement à une interface respiratoire par l'intermédiaire d'un tuyau flexible.

## Patentansprüche

1. Einrichtung zur Atmungsunterstützung, umfassend ein Gehäuse (14), das ein Mikrogebläse (1) umfasst, das einen Elektromotor (2) mit einer drehbaren Motorwelle (3) umfasst, der in einem Kurbelgehäuse (4) angeordnet ist, wobei die Motorwelle (3) mindestens ein Flügelrad (5) trägt, das in einem in einer Spirale (7) gelegenen Innenfach (6) untergebracht ist, wobei das Innenfach (6) einen Gaseinlass (9) und einen Gasauslass (10) umfasst, wobei der Gasauslass (10) in Fluidkommunikation mit einer Gasauslassleitung (11) steht, wobei die Einrichtung weiter ein erstes Dämpfungselement (12) aus flexiblem Material umfasst, das um das Kurbelgehäuse (4) des Motors (2) herum und in Kontakt mit diesem angeordnet ist und dadurch eine Fluidabdichtung zwischen diesen gewährleistet, und ein zweites Dämpfungselement (13) aus flexiblem Material, das um die Auslassleitung (11) herum und in Kontakt mit dieser angeordnet ist und dadurch eine Fluidabdichtung zwischen diesen gewährleistet ist, und **dadurch gekennzeichnet, dass** das zweite Dämpfungselement (13) auf dem ersten Dämpfungselement (12) zur Auflage kommt, und dadurch eine Fluidabdichtung zwischen diesen gewährleistet ist, und das Mikrogebläse (1) durch das erste und das zweite Dämpfungselement (12, 13) in einer festen Position im Gehäuse (14) gehalten wird.

2. Einrichtung nach Anspruch 1, **dadurch gekennzeichnet, dass** das erste Dämpfungselement (12) und/oder das zweite Dämpfungselement (13) aus ElastomerMaterial, bevorzugt Silikon, hergestellt sind.

3. Einrichtung nach einem der vorstehenden Ansprüche, **dadurch gekennzeichnet, dass** das Gehäuse (14) aus mindestens zwei Halbschalen (14a, 14b) gebildet ist, die durch sandwichartiges Einfügen des ersten und/oder des zweiten Dämpfungselements (12, 13) miteinander verbunden sind.

4. Einrichtung nach Anspruch 3, **dadurch gekennzeichnet, dass** das erste Dämpfungselement (12) derart angeordnet ist, dass es eine Fluidabdichtung zwischen den beiden Halbschalen (14a, 14b) des Gehäuses (14) gewährleistet.

5. Einrichtung nach einem der Ansprüche 3 oder 4, **dadurch gekennzeichnet, dass** das zweite Dämpfungselement (13) zwischen einer der beiden Halbschalen (14a) des Gehäuses (14) und dem ersten Dämpfungselement (12) sandwichartig eingefügt ist, und dadurch eine dichte Abdichtung zwischen diesen gewährleistet ist.

6. Einrichtung nach einem der Ansprüche 3 bis 5, **dadurch gekennzeichnet, dass** das Mikrogebläse (1) im Gehäuse durch das erste und zweite Dämpfungselement, bevorzugt nur durch das erste und das zweite Dämpfungselement (12, 13), in Aufhängung gehalten wird.

7. Einrichtung nach einem der Ansprüche 3 bis 6, **dadurch gekennzeichnet, dass** das zweite Dämpfungselement (13) aus einem einzigen Teil gebildet ist, das eine zentrale Öffnung (20) mit kreisförmigem Querschnitt umfasst, und/oder dass das erste Dämpfungselement (12) aus einem einzigen Stück gebildet ist, das eine mehrere Öffnungen (27, 28, 29) umfasst.

8. Einrichtung nach einem der Ansprüche 3 bis 7, **dadurch gekennzeichnet, dass** sich das erste Dämpfungselement (12) zwischen dem Kurbelgehäuse (4) und der Verbindungsstelle der beiden Halbschalen (14a, 14b) des Gehäuses (14) erstreckt.

9. Einrichtung nach einem der Ansprüche 3 bis 8, **dadurch gekennzeichnet, dass** sie weiter mindestens einen zwischen der Spirale (7) und einer der beiden Halbschalen (14a) des Gehäuses (14) angeordneten stoßfesten Anschlag (16), bevorzugt mehrere stoßfeste Anschläge (16), umfasst.

10. Einrichtung nach Anspruch 9, **dadurch gekennzeichnet, dass** die Spirale (7) des Mikrogebläses (1) von einem Pavillon (15) überragt wird, umfassend einen zentralen Durchlass (17), der in Fluidkommunikation mit dem Gaseinlass (9) des Innenraums (6) der Spirale (7) steht, wobei der oder die stoßfesten Anschläge (16) zwischen einer der beiden Halbschalen (14a) des Gehäuses (14) und dem Pavillon (15) angeordnet sind.

11. Anlage zur Ausgabe von Gas, umfassend eine Einrichtung zur Atmungsunterstützung nach einem der vorstehenden Ansprüche, die mittels eines flexiblen Schlauchs mit einer Atmungsschnittstelle fluidisch verbunden ist.

## Claims

1. Breathing assistance apparatus comprising a case (14) comprising a micro-blower (1) comprising an electric motor (2) with a rotating motor shaft (3), arranged in a casing (4), the motor shaft (3) carrying at least one impeller (5) housed in an internal compartment (6) located in a volute (7), said internal compartment (6) comprising a gas inlet (9) and a gas outlet (10), the gas outlet (10) being in fluidic communication with a gas outlet duct (11), said apparatus further comprising a first damping element (12) made of flexible material, arranged around and in contact with the casing (4) of the motor (2) providing a fluidic seal between them, and a second damping element (13) made of flexible material, arranged around and in contact with the outlet duct (11) by providing a fluidic seal between them, and **characterised in that** the second damping element (13) comes to bear against the first damping element (12) by providing a fluidic seal between them, and the micro-blower (1) is maintained in a fixed position in the case (14) by said first and second damping elements (12, 13).

2. Apparatus according to claim 1, **characterised in that** the first damping element (12) and/or the second damping element (13) are made from elastomeric material, preferably silicone.

3. Apparatus according to one of the preceding claims, **characterised in that** the case (14) is formed from at least two half-shells (14a, 14b) that connect to one another sandwiching said first and/or said second damping element (12, 13).

4. Apparatus according to claim 3, **characterised in that** the first damping element (12) is arranged so as to provide a fluidic seal between the two half-shells (14a, 14b) of the case (14).

5. Apparatus according to one of claims 3 or 4, **characterised in that** the second damping element (13) is sandwiched between one of the two half-shells (14a) of the case (14) and the first damping element (12) by providing a fluidic seal between them.

6. Apparatus according to one of claims 3 to 5, **characterised in that** the micro-blower (1) is maintained in suspension in the case by the first and second damping elements, preferably only by said first and second damping elements (12, 13).

7. Apparatus according to one of claims 3 to 6, **characterised in that** the second damping element (13) is formed from a single part comprising a central opening (20) of circular cross-section, and/or the first damping element (12) is formed from a single part comprising several openings (27, 28, 29).

8. Apparatus according to one of claims 3 to 7, **characterised in that** the first damping element (12) extends between the casing (4) and the junction of the two half-shells (14a, 14b) of the case (14).

9. Apparatus according to one of claims 3 to 8, **characterised in that** it further comprises at least one anti-shock abutment (16) arranged between the volute (7) and one of the two half-shells (14a) of the case (14), preferably several anti-shock abutments (16).

10. Apparatus according to claim 9, **characterised in that** the volute (7) of the micro-blower (1) is surmounted with a cowl (15) comprising a central passage (17) in fluidic communication with the gas inlet (9) of the internal compartment (6) of the volute (7), said anti-shock abutment or abutments (16) being arranged between one of the two half-shells (14a) of the case (14) and said cowl (15).

11. Installation of the distribution of gas comprising a breather assistance apparatus according to one of the preceding claims fluidically connected to a breathing interface via a flexible hose.
